# EUROPEAN PATENT APPLICATION

(11) **EP 4 542 227 A1**
(43) Date of publication of application: **23.04.2025**
(21) Application number: 23824268.9
(22) Date of filing: 15.06.2023
(51) Int. Cl.: G01N 33/50

(54) **NOVEL USE OF PBMC-DERIVED CYTOTOXIC T CELLS**

(30) Priority: 15.06.2022 KR 20220073120
(71) Applicant: Organoidsciences Ltd., Seongnam-si, Gyeonggi-do 13493 (KR)
(72) Inventor: LEE, Bo Eun, Seongnam-si Gyeonggi-do 13488 (KR); KIM, Sarang, Seongnam-si Gyeonggi-do 13488 (KR); KIM, Dong Hyeon, Seongnam-si Gyeonggi-do 13488 (KR); LEE, Hyeji, Seongnam-si Gyeonggi-do 13488 (KR); KIM, Eun Jeong, Seongnam-si Gyeonggi-do 13488 (KR)
(74) Representative: Rückerl, Florian
(86) International application number: PCT/KR2023/008325
(87) International publication number: WO 2023/244055

(57) **Abstract**

The present invention relates to: a method for evaluating the efficacy of an anticancer drug using a mixture of peripheral blood mononuclear cells (PBMCs) and cancer organoids; a method for screening the anticancer drug; a system for evaluating the efficacy of the anticancer drug; or a system for screening the anticancer drug. Specifically, the present invention relates to: a method for evaluating the efficacy of an anticancer drug using a mixture of cytotoxic T cells differentiated from PBMCs and cancer organoids; a method for screening the anticancer drug; a system for evaluating the efficacy of the anticancer drug; or a system for screening the anticancer drug. The method for evaluating the efficacy of an anticancer drug, the screening method, the efficacy evaluation system, or the screening system according to the present invention can very closely reproduce a tumor microenvironment, which is the form in which cancer cells exist in a living body. In addition, since PBMCs derived from a normal individual are used through differentiation into cytotoxic T cells specific to a cancer patient, the burden of collecting biological samples such as blood and tissue samples from the cancer patient can be reduced, and the efficacy of an anticancer drug can be evaluated with high accuracy.

## Description

### [Technical Field]

The present invention relates to a method for evaluating the efficacy of an anticancer drug, a method for screening an anticancer drug, a system for evaluating the efficacy of an anticancer drug, or a system for screening an anticancer drug, using a mixture of peripheral blood mononuclear cells (PBMCs) and cancer organoids. Specifically, the present invention relates to a method for evaluating the efficacy of an anticancer drug, a method for screening an anticancer drug, a system for evaluating the efficacy of an anticancer drug, or a system for screening an anticancer drug, using a mixture of cytotoxic T cells differentiated from PBMCs and cancer organoids.

### [Background Art]

An organoid is an organ analogue prepared by three-dimensional culture of cells derived from tissues or organs. It has advantages of long-term culture, cryopreservation, and easy manipulation and observation. In addition, it is used as an experimental model that can study physiological phenomena at a level higher than that of cells by reproducing the hierarchical and histological structures of cells which can be observed in the body, as well as maintaining the original characteristics of the cells without immortalization.

Due to these characteristics, the organoid can evaluate drugs with high accuracy compared to immortalized cell lines with changed intrinsic characteristics thereof or animal models with different structures from the human body. In particular, patient-derived tissues are used, and thus there are advantages in that it is possible to check the efficacy as well as the safety of the drug prior to clinical trials on humans. However, cancer organoids previously developed as drug evaluation models have a limitation in not reflecting the tumor microenvironment, and thus there is a need to develop organoids with various tumor microenvironments and use them as drug evaluation models.

The tumor microenvironment is a cellular environment in which cancer cells are surrounded by blood vessels, immune cells, fibroblasts, lymphocytes, signaling molecules and extracellular matrix (ECM). In the tumor microenvironment, cancer cells influence the microenvironment by inducing extracellular signal release, promotion of cancer cell neovascularization, peripheral immune tolerance, etc. Therefore, cancer cells change the microenvironment, or the microenvironment affects the growth or metastasis of cancer cells. In particular, the tumor microenvironment related to lymphocytes affects not only the occurrence and metastasis of cancer, but also the treatment response by anticancer drugs.

For example, CD8⁺ T cells present in the tumor microenvironment differentiate into cytotoxic T cells, migrate to the tumor microenvironment, and play an important role in tumor immunity, such as killing tumor cells.

### [Technical Problem]

The inventors have conducted various studies to develop cancer organoids with a tumor microenvironment and use them as a platform for drug evaluation or drug screening. As a result, we established a system to co-culture cancer organoids using cytotoxic T cells among the various cells that make up the tumor microenvironment.

In particular, the cytotoxic T cells are obtained by differentiating normal individual-derived PBMCs into cancer patient-specific cytotoxic T cells, thereby reducing the burden of collecting biological samples such as blood and tissue from cancer patients. The present invention was completed by experimentally demonstrating that the cytotoxic T cells maintain survival even after long-term co-culture, thereby enabling long-term evaluation of drug efficacy with high accuracy.

### [Technical Solution]

Each description and embodiment disclosed in the present invention can also be applied to other descriptions and embodiments. That is, all combinations of the various elements disclosed in the present invention fall within the scope of the present invention. Additionally, the scope of the present invention cannot be considered limited by the specific description described below.

In addition, terms that are not specifically defined in this specification should be understood to have meanings commonly used in the technical field to which the present invention pertains. In addition, unless specifically defined by context, the singular includes the plural, and the plural includes the singular.

One aspect of the present invention provides a method for evaluating the efficacy of an anticancer drug, comprising:
(a) mixing and co-culturing peripheral blood mononuclear cells (PBMCs) with cancer organoids to obtain cytotoxic T cells;
(b) mixing and co-culturing the cytotoxic T cells obtained in step (a) with cancer organoids;
(c) treating the mixture of cytotoxic T cells and cancer organoids of step (b) with an anticancer drug; and
(d) determining that the anticancer drug has anticancer efficacy if the group treated with the anticancer drug in step (c) shows increased growth inhibition or death of cancer organoids compared to the group untreated with the anticancer drug or the positive control group.

Specifically, the step (a) is a step of mixing and co-culturing PBMCs and cancer organoids, and differentiates PBMCs into cytotoxic T cells to obtain cytotoxic T cells. In addition, the step (a) is a step of obtaining cytotoxic T cells by differentiating PBMCs derived from normal individuals into cancer patient-specific cytotoxic T cells. In addition, the step (a) is a step in which cancer patient-specific cytotoxic T cells differentiated from PBMC derived from a normal individual learn to recognize cancer cells or cancer organoids of the cancer patient as self cells.

**As** used herein, the term "PBMC" is referred to as "peripheral blood mononuclear cell" and refers to a population of cells composed of monocytes and lymphocytes such as T cells, B cells and NK cells. It is known that in the PBMC population, naïve T cells make up the majority, approximately 45 to 70%, and helper T cells (CD4⁺ T cells) are present at a rate of 25 to 60%, cytotoxic T cells (CD8⁺ T cells) at a rate of 5 to 30%, B cells at a rate of 5 to 10%, NK cells at a rate of 10 to 30%, monocytes at a rate of 5 to 10%, and dendritic cells at a rate of 1 to 2%.

PBMC according to the present invention may be derived from an individual with cancer (cancer patient), a normal individual without cancer, or a normal individual completely cured after developing cancer, and preferably a normal individual without cancer or a normal individual completely cured after developing cancer, but is not limited thereto. In the present specification, the PBMC may be used interchangeably with "pre-differentiated T cells" and the term "derived" may be interchangeably used with "isolated" or "obtained."

**As** used herein, the term "individual" includes all individuals who have never developed cancer, individuals who are likely to develop cancer, individuals who have developed cancer, or individuals who have completely cured after developing cancer, and may include humans or any non-human animals without limitation. The non-human animal may be a vertebrate such as a primate, dog, cow, horse, pig, rodent such as mouse, rat, hamster, guinea pig, etc. In this specification, the term "individual" may be used interchangeably with "subject" or "patient".

**As** used herein, the term "biological sample" may be tissue, cells, whole blood, serum, plasma, or cell culture supernatant, preferably whole blood, but is not limited thereto.

**As** used herein, the term "cytotoxic T cell" refers to a lymphocyte that performs antigen-specific acquired immunity or adaptive immunity. Cytotoxic T cells kill cancer cells or cells infected with pathogens such as viruses and bacteria, and produce cytokines such as TNF-α and IFN-γ.

Cytotoxic T cells according to the present invention may be differentiated from PBMC. In the present specification, the cytotoxic T cells may be used interchangeably with "differentiated T cells," "post-differentiated T cells" or "CD8⁺ T cells".

As used herein, the term "organoid" refers to a mass of cells with a three-dimensional structure, and refers to an organ analogue prepared by three-dimensional culture of cells isolated from an organ or tissue. The organoid can reproduce the particular function and shape of each tissue or organ because it comprises specific cell populations constituting tissues or organs and is structurally organized in a form similar to actual tissues or organs.

**As** used herein, the term "cancer organoid" refers to an organoid prepared from cells (tumor cells or cancer cells) isolated from tumors. The cancer organoid according to the present invention may be derived from an individual with cancer (cancer patient), but is not limited thereto.

In the present invention, the PBMCs and cancer organoids in step (a) may be mixed at a cell number ratio of 15:1 to 25:1, preferably 20:1. Additionally, co-culture in step (a) may be performed for 1 to 42 days. When the PBMCs and cancer organoids are mixed at the ratio and co-cultured for the period of time, PBMCs derived from normal individuals are differentiated into cancer patient-specific cytotoxic T cells, and the differentiation rate increases, so that the burden of collecting biological samples such as blood and tissue from cancer patients can be reduced.

In addition, the step (b) is a step of mixing and co-culturing the cytotoxic T cells (i.e., cytotoxic T cells differentiated from PBMC) obtained in step (a) with cancer organoids, in which the in vivo tumor microenvironment is reproduced in vitro. In addition, the step (b) is a step in which cancer patient-specific cytotoxic T cells differentiated from PBMC derived from normal individuals attack and kill cancer cells or cancer organoids of the cancer patient recognized as self cells.

Herein, the cancer organoids used in step (a) and the cancer organoids used in step (b) may be derived from the same cancer patient.

Additionally, the steps (a) and (b) may be performed continuously in the same culture space. For example, the steps (a) and (b) may be performed sequentially in the same culture plate.

In the present invention, the cytotoxic T cells and cancer organoids in step (b) may be mixed at a cell number ratio of 15:1 to 25:1, preferably 20:1. The cytotoxic T cells do not die even when cultured for a long period of time, and thus can mimic the tumor microenvironment, in which cancer cells exist in vivo, for a long period of time. Accordingly, the efficacy of anticancer drugs can be evaluated with high accuracy for a longer period of time.

In addition, the step (c) is a step of treating the mixture of cytotoxic T cells and cancer organoids of step (b) with an anticancer drug. Specifically, it is the step of treating the mixture of cytotoxic T cells (i.e., cytotoxic T cells differentiated from PBMC) obtained in step (a) and cancer organoids with an anticancer drug.

As used herein, the term "anticancer drug" refers to a substance that has a preventive or therapeutic effect on cancer, specifically, a substance capable of killing cancer cells, cancer organoids or tumors, or inhibiting the growth thereof.

In the present specification, the "anticancer drug" may be used interchangeably with "drug", and the "treating" may be used interchangeably with "adding" or "administering".

Specifically, the anticancer drug may target PBMCs, cytotoxic T cells differentiated from PBMC, cytotoxic T cells, and/or cancer organoids. For example, the anticancer drug may target only cancer organoids, only cytotoxic T cells, or cancer organoids and cytotoxic T cells. For example, an anticancer drug targeting cancer organoids may be atezolizumab, and an anticancer drug targeting cytotoxic T cells may be pembrolizumab.

Preferably, the anticancer drug includes, but not limited to, a compound, a protein, a fusion protein, a compound-protein conjugate, a drug-protein conjugate, an antibody, a compound-antibody conjugate, a drug-antibody conjugate, an amino acid, a peptide, a virus, a carbohydrate, a lipid, a nucleic acid, an extract, and a fraction.

For example, it may include a compound, a peptide, a peptide mimetic, a fusion protein, an antibody, an aptamer and an antibody-drug conjugate (ADC), but is not limited thereto. Preferably, it may be an antibody or an immuno-oncology drug.

**As** used herein, the term "antibody" includes a monoclonal antibody, a polyclonal antibody, a bispecific antibody, a multispecific antibody, a chimeric antibody, a humanized antibody and a human antibody, and also includes antibodies already known in the art or commercially available in addition to novel antibodies. It includes a functional fragment of antibody molecules as well as full-length forms comprising two heavy chains and two light chains. The functional fragment of the antibody molecule refers to a fragment having at least an antigen-binding function, which may include, but not limited to, Fab, F(ab'), F(ab')₂, and Fv. **As** used herein, the term "peptide mimetic" refers to a peptide or a modified peptide that biologically mimics the active ligand of a hormone, a cytokine, an enzyme substrate, a virus or other biological molecule. As used herein, the term "aptamer" refers to a single-stranded nucleic acid (such as DNA, RNA or modified nucleic acid) having a stable tertiary structure and binding to a target molecule with a high affinity and specificity.

As another example, the anticancer drug may include, but not limited to, an antisense nucleic acid, a siRNA, a shRNA, a miRNA and a ribozyme that binds in a complementary manner to a DNA or a mRNA.

As used herein, the term "antisense nucleic acid" refers to DNA, RNA or fragments or derivatives thereof containing a nucleic acid sequence complementary to a particular mRNA sequence, and acts to inhibit the translation of mRNA into protein by binding or hybridizing complementary to the mRNA sequence. As used herein, the term "siRNA (small interfering RNA)" refers to a short double-stranded RNA capable of inducing RNA interference (RNAi) through cleavage of a particular mRNA. The siRNA comprises a sense RNA strand having a sequence homologous to the mRNA of a target gene and an antisense RNA strand having a complementary sequence thereto. Since siRNA can inhibit the expression of a target gene, it is used in a gene knockdown method or a gene therapy method. As used herein, the term "shRNA (short hairpin RNA)", as a single-stranded RNA, refers to an RNA consisting of a stem portion forming a double-stranded portion by hydrogen bonding and a loop portion having a ring shape. It can be processed by a protein such as Dicer and converted into siRNA, and perform the same function as siRNA. As used herein, the term "miRNA (microRNA)" refers to a non-coding RNA of 21 to 23 nt that regulates gene expression after transcription by promoting degradation of target RNA or inhibiting translation thereof. As used herein, the term "ribozyme" refers to an RNA molecule having an enzyme-like function that recognizes a particular nucleotide sequence and cuts it by itself. The ribozyme consists of a region that binds specifically by a complementary nucleotide sequence of a target messenger RNA strand and a region that cuts the target RNA.

The anticancer drug to be evaluated or screened according to the method of the present invention may be, for example, preventing or treating biliary tract cancer, stomach cancer, lung cancer, liver cancer, colorectal cancer, colon cancer, small intestine cancer, pancreatic cancer, brain cancer, osteosarcoma, melanoma, breast cancer, sclerosing adenosis, uterine cancer, cervical cancer, head and neck cancer, esophageal cancer, thyroid cancer, parathyroid cancer, renal cancer, sarcoma, prostate cancer, urethral cancer, bladder cancer, hematologic malignancy, leukemia, lymphoma and fibroadenoma, but is not limited thereto.

In addition, the step (d) is a step of determining that the anticancer drug has anticancer efficacy when a growth inhibition or death of cancer organoids is increased in the group treated with the anticancer drug in step (c) compared to a positive control group or a group not treated with the anticancer drug.

**As** used herein, the term "positive control group" refers to a group treated with a substance used as an anticancer drug in the art.

Specifically, the growth inhibition or death of cancer organoids may be confirmed by the increase or decrease of the volume, area or cell number of the cancer organoids. When the volume, area or cell number of the cancer organoids is decreased, it may be determined that growth inhibition or death of the cancer organoids is increased. The increase or decrease in the volume, area or cell number of the cancer organoids may be confirmed by a method known in the art, for example, but not limited thereto, an analysis method such as High-Content Screening (HCS) or Flow-cytometer.

Another aspect of the present invention provides a method for screening an anticancer drug, comprising:
(a) mixing and co-culturing PBMCs with cancer organoids to obtain cytotoxic T cells;
(b) mixing and co-culturing the cytotoxic T cells obtained in step (a) with cancer organoids;
(c) treating the mixture of cytotoxic T cells and cancer organoids of step (b) with an anticancer drug candidate; and
(d) determining the anticancer drug candidate as an anticancer drug if the group treated with the anticancer drug candidate in step (c) shows increased growth inhibition or death of cancer organoids compared to the group untreated with the anticancer drug candidate.

In the method for screening an anticancer drug according to the present invention, unless specifically stated otherwise, related terms are understood to have the same meaning as the terms described above.

Specifically, the step (a) is a step of mixing and co-culturing PBMCs and cancer organoids, and differentiates PBMCs into cytotoxic T cells to obtain cytotoxic T cells. In addition, the step (a) is a step of obtaining cytotoxic T cells by differentiating PBMCs derived from normal individuals into cancer patient-specific cytotoxic T cells. In addition, the step (a) is a step in which cancer patient-specific cytotoxic T cells differentiated from PBMC derived from a normal individual learn to recognize cancer cells or cancer organoids of the cancer patient as self cells.

In the present invention, the PBMCs and cancer organoids in step (a) may be mixed at a cell number ratio of 15:1 to 25:1, preferably 20:1. Additionally, co-culture in step (a) may be performed for 1 to 42 days. When the PBMCs and cancer organoids are mixed at the ratio and co-cultured for the period of time, PBMCs derived from normal individuals are differentiated into cancer patient-specific cytotoxic T cells, and the differentiation rate increases, so that the burden of collecting biological samples such as blood and tissue from cancer patients can be reduced.

In addition, the step (b) is a step of mixing and co-culturing the cytotoxic T cells (i.e., cytotoxic T cells differentiated from PBMC) obtained in step (a) with cancer organoids, in which the in vivo tumor microenvironment is reproduced in vitro. In addition, the step (b) is a step in which cancer patient-specific cytotoxic T cells differentiated from PBMC derived from normal individuals attack and kill cancer cells or cancer organoids of the cancer patient recognized as self cells.

Herein, the cancer organoids used in step (a) and the cancer organoids used in step (b) may be derived from the same cancer patient.

Additionally, the steps (a) and (b) may be performed continuously in the same culture space. For example, the steps (a) and (b) may be performed sequentially in the same culture plate.

In the present invention, the PBMCs and cancer organoids in step (b) may be mixed at a cell number ratio of 15:1 to 25:1, preferably 20:1. The cytotoxic T cells do not die even when cultured for a long period of time, and thus can mimic the tumor microenvironment, in which cancer cells exist in vivo, for a long period of time. Accordingly, the efficacy of anticancer drugs can be evaluated with high accuracy for a longer period of time.

In addition, the step (c) is a step of treating the mixture of cytotoxic T cells and cancer organoids of step (b) with an anticancer drug candidate. Specifically, it is the step of treating the mixture of cytotoxic T cells (i.e., cytotoxic T cells differentiated from PBMC) obtained in step (a) and cancer organoids with an anticancer drug candidate.

**As** used herein, the term "anticancer drug candidate" refers to a substance that is expected to have a preventive or therapeutic effect on cancer, specifically, a substance that is expected to kill cancer cells, cancer organoids or tumors, or inhibit the growth thereof.

In the present specification, the "anticancer drug candidate" may be used interchangeably with 'drug', and the "treating" may be used interchangeably with "adding" or "administering".

Preferably, the anticancer drug candidate includes, but not limited to, a compound, a protein, a fusion protein, a compound-protein conjugate, a drug-protein conjugate, an antibody, a compound-antibody conjugate, a drug-antibody conjugate, an amino acid, a peptide, a virus, a carbohydrate, a lipid, a nucleic acid, an extract, and a fraction.

For example, it may include a compound, a peptide, a peptide mimetic, a fusion protein, an antibody, an aptamer and an antibody-drug conjugate (ADC), but is not limited thereto. Preferably, it may be an antibody or an immuno-oncology drug.

In addition, the step (d) is a step of determining the anticancer drug candidate as an anticancer drug if the group treated with the anticancer drug candidate in step (c) shows increased growth inhibition or death of cancer organoids compared to the group untreated with the anticancer drug candidate.

Specifically, the growth inhibition or death of cancer organoids may be confirmed by the increase or decrease of the volume, area or cell number of the cancer organoids. When the volume, area or cell number of the cancer organoids is decreased, it may be determined that growth inhibition or death of the cancer organoids is increased. The increase or decrease in the volume, area or cell number of the cancer organoids may be confirmed by a method known in the art, for example, but not limited thereto, an analysis method such as High-Content Screening (HCS) or Flow-cytometer.

Another aspect of the present invention is an anticancer drug efficacy evaluation system for using the method for evaluating the efficacy of an anticancer drug, and provides an anticancer drug efficacy evaluation system comprising PBMCs and cancer organoids.

In the efficacy evaluation system for anticancer drug according to the present invention, unless specifically stated otherwise, related terms are understood to have the same meaning as the terms described above.

The efficacy evaluation system for anticancer drug according to the present invention comprises PBMCs and cancer organoids. Even though the PBMCs are derived from normal individuals, they exhibit the characteristic of differentiating into cancer patient-specific cytotoxic T cells. Therefore, the burden of collecting biological samples such as blood and tissue from cancer patients can be reduced. In addition, the cytotoxic T cells differentiated from PBMCs do not die even when cultured with cancer organoids for a long period of time, and thus they can mimic the tumor microenvironment, in which cancer cells exist in vivo, for a long period of time. Therefore, the efficacy of an anticancer drug when administered in vivo can be accurately predicted, and furthermore, the efficacy of an anticancer drug can be evaluated with high accuracy over a longer period of time. Accordingly, it can be usefully used to evaluate the efficacy of an anticancer drug.

Another aspect of the present invention is an anticancer drug screening system for using the method for screening an anticancer drug, and provides an anticancer drug screening system comprising PBMCs and cancer organoids.

In the screening system for anticancer drug according to the present invention, unless specifically stated otherwise, related terms are understood to have the same meaning as the terms described above.

The screening system for anticancer drug according to the present invention comprises PBMCs and cancer organoids. Even though the PBMCs are derived from normal individuals, they exhibit the characteristic of differentiating into cancer patient-specific cytotoxic T cells. Therefore, the burden of collecting biological samples such as blood and tissue from cancer patients can be reduced. In addition, the cytotoxic T cells differentiated from PBMCs do not die even when cultured with cancer organoids for a long period of time, and thus they can mimic the tumor microenvironment, in which cancer cells exist in vivo, for a long period of time. Therefore, the efficacy of an anticancer drug when administered in vivo can be accurately predicted, and furthermore, the efficacy of an anticancer drug can be evaluated with high accuracy over a longer period of time. Accordingly, it can be usefully used for screening an anticancer drug.

The method for evaluating the efficacy of an anticancer drug, the screening method, the efficacy evaluation system, or the screening system according to the present invention can very closely reproduce a tumor microenvironment, which is the form in which cancer cells exist in a living body. In addition, since PBMCs derived from a normal individual are used through differentiation into cytotoxic T cells specific to a cancer patient, the burden of collecting biological samples such as blood and tissue samples from the cancer patient can be reduced, and the efficacy of an anticancer drug can be evaluated with high accuracy.

### [Brief Description of Figures]

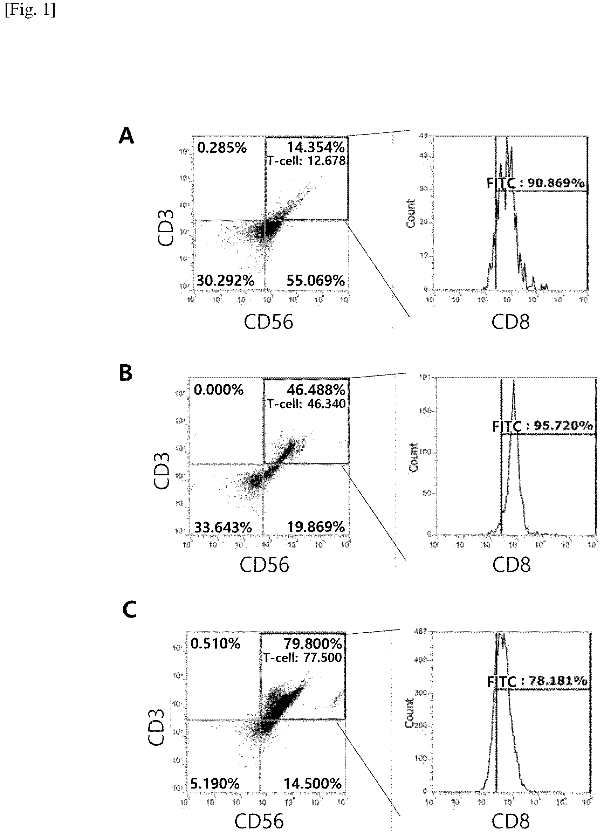

FIG. 1 shows the results of FACS analysis on peripheral blood mononuclear cells (PBMCs) co-cultured with cancer organoids according to the first step, where A is a chromatogram for Matching condition, B is a chromatogram for Non-matching condition, and C is a chromatogram for Normal condition. After gating for CD56⁺CD3⁺, gating was performed for CD8⁺.

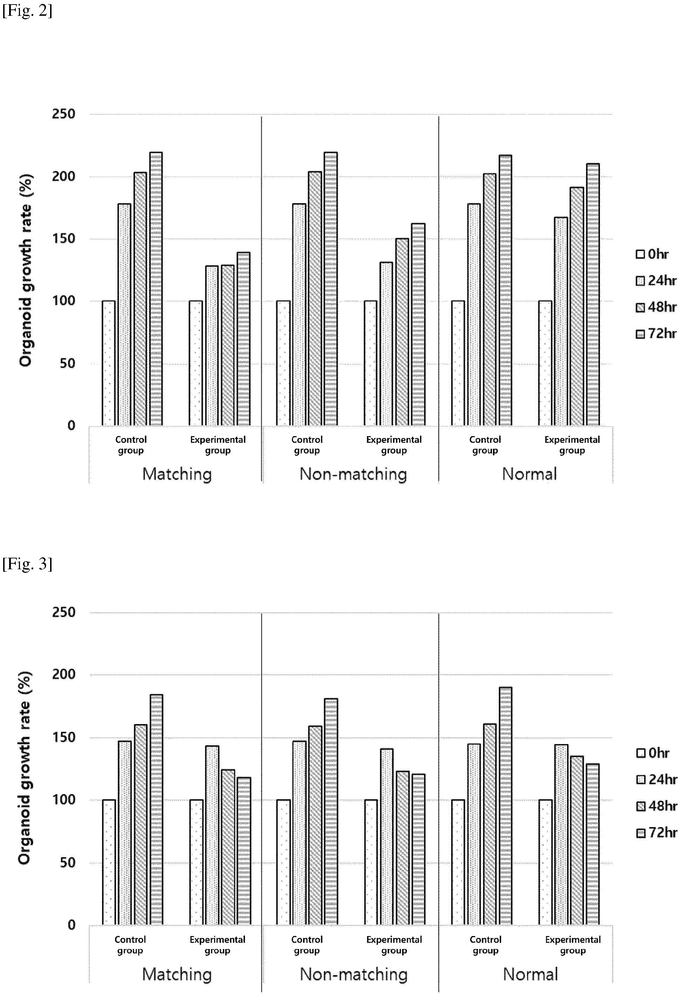

FIG. 2 is a graph showing the growth rate of cancer organoids as a result of treatment with atezolizumab as a drug on the 14th day after the start of co-culture in the first step. The Control group refers to the untreated group, and the Experimental group refers to the group treated with 10 nM of atezolizumab.
FIG. 3 is a graph showing the growth rate of cancer organoids as a result of treatment with atezolizumab as a drug on the 28th day after the start of co-culture in the first step. The Control group refers to the untreated group, and the Experimental group refers to the group treated with 10 nM of atezolizumab.

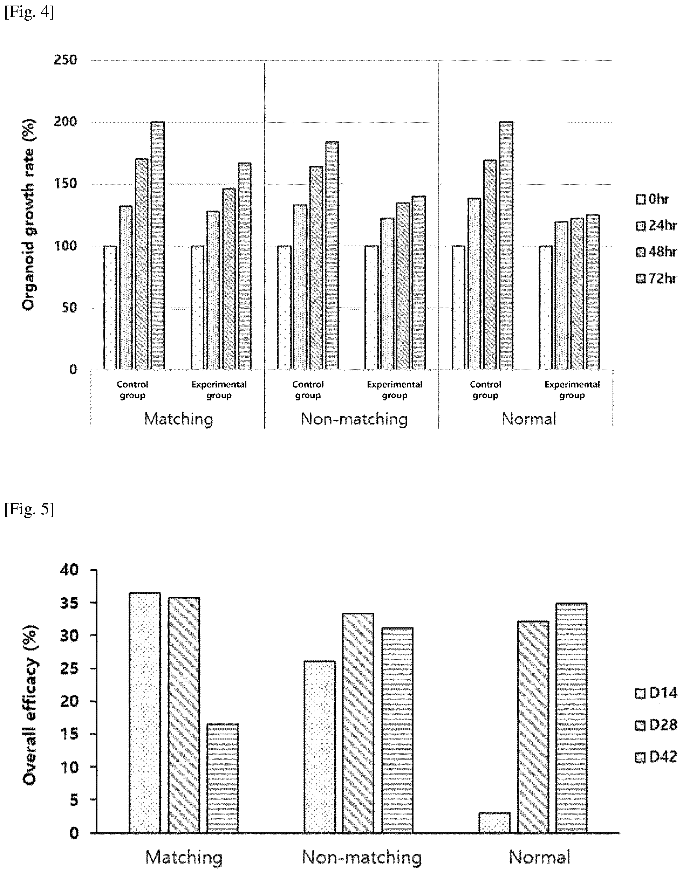

FIG. 4 is a graph showing the growth rate of cancer organoids as a result of treatment with atezolizumab as a drug on the 42nd day after the start of co-culture in the first step. The Control group refers to the untreated group, and the Experimental group refers to the group treated with 10 nM of atezolizumab.
FIG. 5 is a graph showing the overall efficiency of atezolizumab as a result of treatment with atezolizumab on the 14th, 28th or 42nd day after the start of co-culture in the first step.

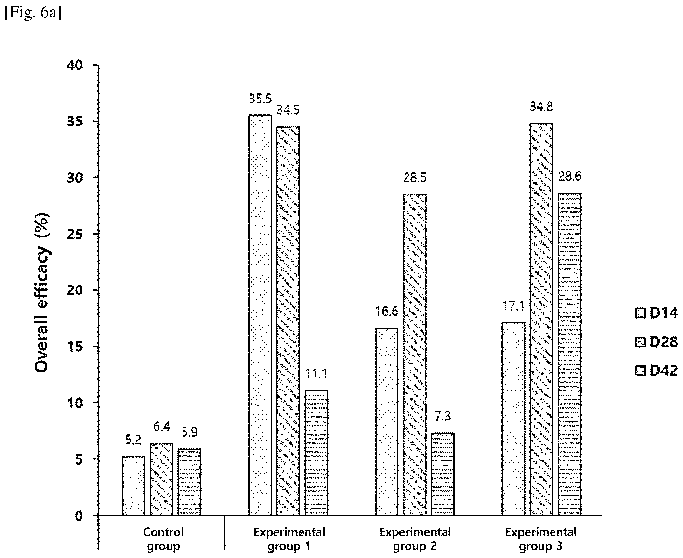

FIG. 6a is a graph showing the overall efficiency of atezolizumab as a result of treatment with atezolizumab on the 14th, 28th or 42nd day after the start of co-culture in the first step, respectively, in the Control group and Experimental groups 1 to 3.

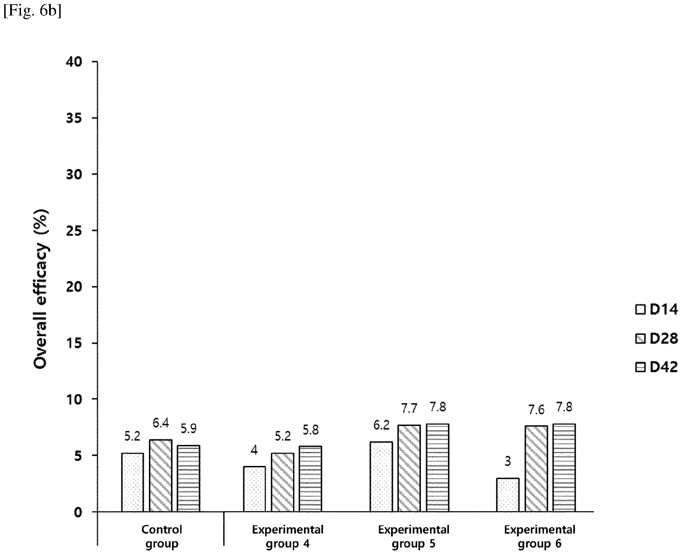

FIG. 6b is a graph showing the overall efficiency of atezolizumab as a result of treatment with atezolizumab on the 14th, 28th or 42nd day after the start of co-culture in the first step, respectively, in the Control group and Experimental groups 4 to 6.

### [Examples]

Hereinafter, the present invention will be explained in more detail through examples. These examples are for illustrating the present invention more specifically, and the scope of the present invention is not limited by these examples.

### Example 1. Establishment of Anticancer Drug Efficacy Evaluation and Screening System

The inventors have established an *in vitro* efficacy evaluation and screening system that can accurately predict the efficacy of a drug when administered to a subject, by similarly reproducing the tumor microenvironment in which cancer exists in the body.

Specifically, a co-culture system in which cancer organoids and T cells coexist was used. The cancer organoids and T cells may be derived from various individuals, such as an individual with cancer (cancer patient), a normal individual without cancer, or a normal individual cured after developing cancer. Depending on their source, the co-culture system may be divided into Matching, Non-matching and Normal conditions. The Matching condition uses cancer organoids and T cells derived from the same cancer patient, for example, cancer organoids derived from cancer patient A and T cells derived from cancer patient A. The Non-matching condition uses cancer organoids and T cells derived from different cancer patients, for example, cancer organoids derived from cancer patient A and T cells derived from cancer patient B. The Normal condition uses cancer organoids derived from cancer patients and T cells derived from normal individual, for example, cancer organoids derived from cancer patient A and T cells derived from normal individual C.

The Matching and Non-matching conditions have the advantage of being able to more closely in vitro reproduce the in vivo tumor microenvironment, in that both cancer organoids and T cells are derived from individuals that have already developed cancer. However, a process of collecting blood samples from an individual is necessary in order to isolate T cells, and collecting blood samples from cancer patients has a negative impact on the improvement of cancer patients' condition.

Therefore, for the purpose of reducing the burden of collecting biological samples such as blood and tissue from cancer patients, we established a co-culture system under Normal condition that can reproduce the tumor microenvironment to a degree similar to or better than Matching or Non-matching conditions.

Specifically, the system under Normal condition was prepared through a first step of co-culturing peripheral blood mononuclear cells (PBMCs) derived from normal individuals and cancer organoids derived from cancer patients to differentiate the PBMCs into T cells; a second step of co-culturing the differentiated T cells with cancer organoids derived from patients.

More specifically, the majority of PBMCs isolated from normal individuals are naive T cells, and differentiated cytotoxic T cells that can attack cancer cells are present at a very low rate. Therefore, in order to mimic an in vivo tumor microenvironment, PBMCs were differentiated into cytotoxic T cells by providing cancer organoids as antigens to PBMCs. For PBMCs derived from normal individuals, cancer organoids derived from cancer patients are provided as antigens to differentiate PBMCs into cytotoxic T cells, and also the cytotoxic T cells derived from normal individuals were able to recognize cancer organoids derived from cancer patients as self cells.

This was accomplished through the first step of co-culturing cancer organoids and PBMCs. Cancer organoids and PBMCs were manufactured and prepared as follows.

Lung cancer organoids were used as the type of cancer organoid. Organoids with a 3D structure were prepared by separating lung cancer tissue obtained from a patient into single cells and culturing them with extracellular matrix (ECM). The formed lung cancer organoid was confirmed to be a tumor through pathologic analysis, and was separated into single cells using Tryp-LE before use.

PBMCs were separated from blood obtained from donors using Ficoll. **After** separation, they were stored in a nitrogen tank until use. They were thawed immediately before use, stabilized for one day, and then used.

Afterwards, the first step was performed by mixing single cell lung cancer organoids and PBMCs at a cell number ratio of 1:20 (7.5×10³ cell/well: 1.5×10⁵ cell/well) and culturing for 0 to 42 days. The culture period was continued while checking using a flow cytometer until the expression of cytotoxic T cells was greater than 10%. Afterwards, the second step was performed by mixing the cytotoxic T cells obtained by the above method with the cancer patient-derived cancer organoids at a cell number ratio of 1:20, and further culturing them. Through this, we established a system that mimics the in vivo tumor microenvironment in which cytotoxic T cells kill cancer organoids.

### Example 2. Verification of Anticancer Drug Efficacy Evaluation and Screening System

### Example 2.1. Confirmation of cytotoxic T cell marker expression

In order to verify whether the system established in Example 1 can be appropriately used for drug efficacy evaluation and screening, it was analyzed whether the differentiation of PBMC into cytotoxic T cells by performing the first step progressed well.

Specifically, the proportion of cells expressing CD56, CD3 and CD8 proteins on the cell surface as cytotoxic T cell markers was analyzed. FACS analysis was performed on the PBMCs co-cultured in the first step under each of Matching, Non-matching and Normal condition, and the proportion of CD56⁺CD3⁺CD8⁺ T cells present in the PBMC population was analyzed.

**Table 1**

| | Day 0 | Day 14 | Day 28 | Day 42 |
|---|---|---|---|---|
| Matching | 12.8% | 42.5% | 60.4% | 13.1% |
| Non-matching | 12.6% | 46.4% | 53.7% | 44.5% |
| Normal | 0.3% | 9.6% | 50.8% | 62.4% |

The results are shown in Table 1 and FIG. 1,
Under Matching condition, cytotoxic T cells increased rapidly from day 0 to day 28 in the first stage of co-culture, and about 60.4% of cytotoxic T cells were present on day 28. However, during long-term co-culture after day 42, it was confirmed that most of the cytotoxic T cells had died, returning to about 13.1%, the same level as on day 0 from the start of co-culture.

Under Non-matching condition, cytotoxic T cells increased rapidly from day 0 to day 28 in the first stage of co-culture, and about 53.7% of cytotoxic T cells were present on day 28. It was confirmed that cytotoxic T cells maintained a differentiation level that is not low, at approximately 44.5%, even during long-term co-culture after day 42.

Under Normal condition, cytotoxic T cells were nearly absent from day 0 to day 14 in the first stage of co-culture. However, from day 28, cytotoxic T cells rapidly increased and were maintained at a high level of approximately 50%. In particular, during long-term co-culture after day 42, cytotoxic T cells showed a significantly high level of approximately 62.4%, and it was confirmed that the level is higher than the maximum under Matching condition (60.4% on day 28) and is about 4.7 times higher than 13.1% on day 42.

These results suggest that when PBMCs are differentiated into cytotoxic T cells according to the method of Example 1 (first stage) and an in vitro system that mimics the tumor microenvironment is prepared using the cytotoxic T cells (second stage), PBMCs isolated from normal individuals can be differentiated into cytotoxic T cells at a level similar to that of PBMCs isolated from cancer patients, and thus a system to be used for drug screening and efficacy evaluation can be prepared in an efficient manner to more closely resemble the in vivo tumor microenvironment.

### Example 2.2. Confirmation of interferon-producing cell (ICP) marker expression

In order to verify whether the system established in Example 1 can be appropriately used for drug efficacy evaluation and screening, it was analyzed whether the differentiation of PBMC into cytotoxic T cells by performing the first step progressed well.

Specifically, cytotoxic T cells express ImmuneCheck Point Protein (ICP), and the expression level is known to vary depending on each person. We analyzed whether the ICP marker was expressed equally in the cytotoxic T cells under Matching conditions and under Normal differentiation conditions. It was analyzed to see if it was possible. FACS analysis was performed on the cytotoxic T cells for which differentiation and separation were completed according to Example 1.1. Then, among the cytotoxic T cells, the proportion (indicated as % in Table 2) and number (indicated in parentheses in Table 2) of cytotoxic T cells expressing LAG3, TIM3, PD-1, Light, 4-1BB or BTLA, known as ICP markers, on the cell surface were analyzed.

**Table 2**

| | Matching | Non-matching | Normal |
|---|---|---|---|
| LAG3 | 96.0% (499) | 94.5% (431) | 95.7% (367) |
| TIM3 | 98.1% (986) | 98.0% (1007) | 97.7% (976) |
| PD-1 | 94.5% (465) | 92.2% (172) | 92.8% (92) |
| Light | 99.1% (2062) | 98.3% (2378) | 98.8% (1875) |
| 4-1BB | 97.8% (301) | 95.5% (405) | 96.8% (186) |
| BTLA | 98.8% (202) | 98.2% (250) | 98.8% (181) |

**As** a result, as shown in Table 2, in each Matching, Non-matching and Normal condition, cytotoxic T cells all expressed ICP markers at similar levels.

These results suggest that when PBMCs are differentiated into cytotoxic T cells according to the method of Example 1 (first stage) and a system is prepared using the cytotoxic T cells (second stage), PBMCs isolated from normal individuals can be differentiated into cytotoxic T cells at a level similar to that of PBMCs isolated from cancer patients, and thus a system to be used for drug screening and efficacy evaluation can be prepared in an efficient manner to more closely resemble the in vivo tumor microenvironment.

### Example 2.3. Evaluation of Immuno-oncology Drug Efficacy

### Example 2.3.1. Efficacy evaluation depending on culture period

In order to verify whether the system established in Example 1 can be appropriately used for drug efficacy evaluation and screening, the system prepared according to the first and second steps was treated with atezolizumab as a drug, and then we evaluated its ability to kill cancer organoids. Atezolizumab is a drug currently marketed and used as a treatment for lung cancer.

Specifically, the system performed the first step of co-culture for 42 days. 10 nM of atezolizumab was treated on the 14th, 28th and 42nd days of co-culture, respectively, and the efficacy of drug treatment for 72 hours was evaluated. Afterwards, the growth rate of cancer organoids was determined at 0H, 12H, 24H and 72H after atezolizumab treatment, respectively, to analyze the cancer organoids growth inhibition (killing of cancer organoids) effect of atezolizumab and overall efficacy. The overall efficacy was evaluated by calculating the change in organoid area of the drug-treated group compared to the organoid area of the drug-untreated group 72 hours after drug treatment.

The results are shown in FIGs. 2 to 4.

On the 14th day from the start of the first step of co-culture, the effect of atezolizumab on inhibiting the growth of cancer organoids (killing cancer organoids) was shown to be the best under Matching condition, and under Non-matching condition, the effect was lower than Matching condition. Under Normal condition, almost no effect was observed, showing a growth rate similar to that of the Control group (FIG. 2).

On the other hand, on the 28th day from the start of the first step of co-culture, the effect of atezolizumab on the growth inhibition of cancer organoids (killing cancer organoids) was shown to be the best under Matching condition, but Non-matching or Normal condition also showed a similar effect to Matching condition (FIG. 3).

In particular, on the 42nd day from the start of the first step of co-culture, the effect of atezolizumab on inhibiting the growth of cancer organoids (killing cancer organoids) was the lowest under Matching condition, and was remarkably excellent under Normal condition. The effect of Non-matching condition was lower than that of Normal condition (FIG. 4).

### In light of this, as shown in FIG. 5,

Under Matching condition, the overall efficacy of atezolizumab is maintained at about 35% to 36% from day 0 to day 28 of the first step of co-culture. However, during long-term culture after day 42, the effect sharply decreased to about 16%, which corresponds to half of the effect.

Under Non-matching condition, the overall efficacy of atezolizumab remained similar at about 26 to 33% from day 0 to day 42 of the first step of co-culture.

Under Normal condition, the overall efficacy of atezolizumab was barely shown at about 3% from day 0 to day 14 of the first step of co-culture. However, it rapidly increased to about 32% from day 28, and the effect was further improved to about 35% during long-term co-culture after day 42. This effect was superior to the maximum effect (about 33%) in Non-matching condition, and similar to the maximum effect (about 36%) in Matching condition.

These results indicate that the system prepared by the method according to Example 1 mimics the tumor microenvironment and can be usefully used for drug screening and efficacy evaluation.

In particular, it shows that under Normal condition, PBMCs from normal individuals are differentiated into cancer patient-specific cytotoxic T cells, which has the advantage of not requiring additional biological samples to be collected from cancer patients and evaluating drug efficacy with high accuracy even during long-term co-culture. Accordingly, it suggests that it can be useful for screening and efficacy evaluation of new drugs with increased half-life.

### Example 2.3.2. Efficacy Evaluation under Normal Condition

It was verified whether the co-culture system under Normal condition established in Example 1 could be appropriately used for drug efficacy evaluation and drug screening.

Specifically, each cancer organoids derived from different cancer patients (patient A, patient B) was used to differentiate PBMCs derived from a normal individual (normal subject C) into cytotoxic T cells (first step). The origins of PBMCs and cancer organoids used in each experimental group are listed in Table 3.

**Table 3**

| | Origin of PBMCs | Origin of cancer organoids | Condition |
|---|---|---|---|
| Control group | - | Patient A | - |
| Experimental group 1 | Patient A | Patient A | Matching condition |
| Experimental group 2 | Patient B | Patient A | Non-matching condition |
| Experimental group 3 | Normal subject C | Patient A | Normal condition |
| Experimental group 4 | Patient A | Patient B | Non-matching condition |
| Experimental group 5 | Patient B | Patient B | Matching condition |
| Experimental group 6 | Normal subject C | Patient B | Normal condition |

Cytotoxic T cells differentiated in each experimental group were mixed with cancer organoids derived from patient A and co-cultured (second step). The co-culture was treated with atezolizumab by the method according to Example 2.3.1., and then its efficacy was evaluated.

The results are shown in FIG. 6a.

In Experimental groups 1 to 3 (cancer organoids derived from the same cancer patient A were used in the first and second steps), it was confirmed that the overall efficacy of atezolizumab was up to approximately 5 times higher compared to that in the Control group in which only cancer organoids were present. In particular, in Experimental group 3 (Normal condition in which normal subject C-derived PBMCs were differentiated into cytotoxic T cells for patient A), it was confirmed that the overall efficacy of atezolizumab increased rapidly from day 15 of the first step of co-culture, reaching about 35%, and the effect remained high at about 30% even during long-term co-culture after day 42.

On the other hand, as shown in FIG. 6b,
It was confirmed that in Experimental groups 4 to 6 (cancer organoids derived from different cancer patients were used in the first and second steps. That is, the first step used cancer organoids derived from cancer patient B, and the second step used cancer organoids derived from cancer patient A), the overall efficacy of atezolizumab was similar to that in the Control group in which only cancer organoids were present. In particular, in Experimental group 6 (Normal condition in which normal subject-derived PBMCs were differentiated into cytotoxic T cells), it was confirmed that the overall efficacy of atezolizumab was not significantly different compared to that in the Control group.

These results show that, under co-culture conditions using PBMCs derived from the same normal individual, the system can be useful for drug screening and efficacy evaluation only when the cancer organoids used in the first and second steps are derived from the same cancer patient.

## Claims

1. A method for evaluating the efficacy of an anticancer drug, comprising:
(a) mixing and co-culturing peripheral blood mononuclear cells (PBMCs) with cancer organoids to obtain cytotoxic T cells;
(b) mixing and co-culturing the cytotoxic T cells obtained in step (a) with cancer organoids;
(c) treating the mixture of cytotoxic T cells and cancer organoids of step (b) with an anticancer drug; and
(d) determining that the anticancer drug has anticancer efficacy if the group treated with the anticancer drug in step (c) shows increased growth inhibition or death of cancer organoids compared to the group untreated with the anticancer drug or the positive control group.

2. A method for screening an anticancer drug, comprising:
(a) mixing and co-culturing PBMCs with cancer organoids to obtain cytotoxic T cells;
(b) mixing and co-culturing the cytotoxic T cells obtained in step (a) with cancer organoids;
(c) treating the mixture of cytotoxic T cells and cancer organoids of step (b) with an anticancer drug candidate; and
(d) determining the anticancer drug candidate as an anticancer drug if the group treated with the anticancer drug candidate in step (c) shows increased growth inhibition or death of cancer organoids compared to the group untreated with the anticancer drug candidate.

3. The method according to claim 1 or 2, wherein the PBMCs in step (a) are derived from a normal individual.

4. The method according to claim 1 or 2, wherein the cancer organoids of step (a) are derived from a cancer patient.

5. The method according to claim 1 or 2, wherein the cancer organoids in step (a) and the cancer organoids in step (b) are derived from the same cancer patient.

6. The method according to claim 1 or 2, wherein the co-culturing in step (a) is performed for 14 to 42 days.

7. The method according to claim 1 or 2, wherein the anticancer drug is at least one selected from the group consisting of a compound, a peptide, a peptide mimetic, a fusion protein, an antibody, an aptamer, an antibody-drug conjugate (ADC), and an antisense nucleic acid, a siRNA, a shRNA, a miRNA and a ribozyme that binds in a complementary manner to a DNA or a mRNA.

8. The method according to claim 1 or 2, wherein the cancer is at least one selected from the group consisting of biliary tract cancer, stomach cancer, lung cancer, liver cancer, colorectal cancer, colon cancer, small intestine cancer, pancreatic cancer, brain cancer, osteosarcoma, melanoma, breast cancer, sclerosing adenosis, uterine cancer, cervical cancer, head and neck cancer, esophageal cancer, thyroid cancer, parathyroid cancer, renal cancer, sarcoma, prostate cancer, urethral cancer, bladder cancer, hematologic malignancy, lymphoma and fibroadenoma.

9. The method according to claim 1 or 2, wherein the growth inhibition or death of the cancer organoids in step (d) is confirmed through an increase or decrease in the volume, area or cell number of the cancer organoids.

10. A system for evaluating the efficacy of an anticancer drug to use the method for evaluating the efficacy of an anticancer drug according to claim 1, comprising PBMCs and cancer organoids.

11. A system for screening an anticancer drug to use the method for screening an anticancer drug according to claim 2, comprising PBMCs and cancer organoids.
